# EUROPEAN PATENT SPECIFICATION

(11) **EP 3 505 507 B1**
(45) Date of publication and mention of the grant of the patent: **27.05.2020**
(21) Application number: 19150060.2
(22) Date of filing: 02.01.2019
(51) Int. Cl.: C07C 37/82, C07C 39/14, B01D 15/08, B01J 20/08

(54) **METHOD FOR PURIFYING DIHYDROXYNAPHTHALENE**
VERFAHREN ZUR REINIGUNG VON DIHYDROXYNAPHTHALEN
PROCÉDÉ DE PURIFICATION DE DIHYDROXYNAPHTHALÈNE

(30) Priority: 26.12.2017 JP 2017248936
(43) Date of publication of application: 03.07.2019
(73) Proprietor: Shin-Etsu Chemical Co., Ltd., Chiyoda-ku, Tokyo (JP)
(72) Inventor: Tachibana, Seiichiro, Niigata (JP); Kori, Daisuke, Niigata (JP); Ogihara, Tsutomu, Niigata (JP); Kitano, Satoru, Gunma (JP); Abe, Yukio, Gunma (JP); Hatakeyama, Fumihiro, Gunma (JP); Kobayashi, Taiki, Gunma (JP)
(74) Representative: Wibbelmann, Jobst

(56) References cited:
- WO-A1-2017/038968
- CN-A- 102 442 889
- JP-A- H0 987 220
- JP-A- S6 339 831
- JP-A- H09 208 518

## Description

### TECHNICAL FIELD

The present invention relates to a method for purifying dihydroxynaphthalene.

### BACKGROUND ART

Dihydroxynaphthalene resins are widely used in the semiconductor field as materials for underlayer films, photoresists, and the like because of the excellent heat resistance and moisture resistance (Patent Literature 1).

Common dihydroxynaphthalene (1) is generally produced by the following method in an industrial scale. Specifically, the starting material naphthalene (1-1) is sulfonated to form a sulfonic acid compound (1-2). Then, this compound is converted to have hydroxyl groups by alkali fusion, so that the dihydroxynaphthalene (1) is obtained. 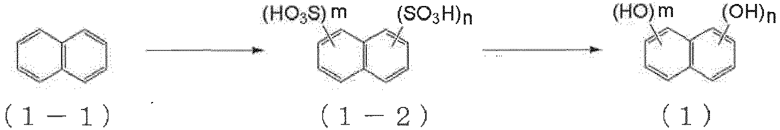 In the formulae, "n" and "m" represent integers satisfying 0≤m≤2, 0≤n≤2, and m+n=2.

In this production process, the sulfonic acid compound (1-2) is not completely consumed by the alkali fusion. As a sulfur element content among the constituents elements, the sulfonic acid compound (1-2) in an amount of approximately several hundred ppm to several thousand ppm in terms of mass remains in the dihydroxynaphthalene (1) of general industrial grades (hereinafter, the sulfonic acid compound is also referred to as "sulfur content"). Heretofore, industrial-grade dihydroxynaphthalenes containing such impurities have been mainly used as dyes. Hence, such impurities have not brought about any problems.

Generally, when a composition for forming an organic film for manufacturing a semiconductor device is used in the process of manufacturing a semiconductor device, the composition has to be purified by precise filtration using a filter having fine openings so as to eliminate defect in coating film or defect after dry etching. If this purification operation is insufficient, an electronic circuit in the semiconductor device malfunctions due to the defect in coating film or defect after dry etching, and the yield in manufacturing a semiconductor device is decreased. To prevent such a yield decrease in manufacturing a semiconductor device, the difference in the hydraulic pressure of the composition before and after the filter needs to be precisely controlled for the precise filtration.

When a dihydroxynaphthalene resin produced by using conventionally known industrial-grade dihydroxynaphthalene as the starting material is used to produce a composition for forming an organic film for manufacturing a semiconductor device, clogging occurs in the purification step by precise filtration with a fine filter having openings of 20 nm or less, which is essential for the most advanced processing material for manufacturing a semiconductor device. Hence, it has been difficult to adopt the resin as the processing material for manufacturing a semiconductor device.

Such a dihydroxynaphthalene resin presumably contains hard foreign matters (hereinafter referred to as hard particles) and soft foreign matters (hereinafter referred to as soft particles) which are deformable by a weak force due to solvent incorporation. To adopt such a dihydroxynaphthalene resin solution or a composition containing this resin (hereinafter referred to as dihydroxynaphthalene composition) as a composition for manufacturing a semiconductor device, purification with a fine filter is necessary. For example, when precise filtration is carried out using a fine filter for the purification by removing hard particles in a dihydroxynaphthalene composition, the hard particles can be captured on the filter surface. Even when the hard particles are captured on the filter surface, the passage amount during the filtration hardly changes owing to gaps among the hard particles, so that the ability to purify the dihydroxynaphthalene resin composition is maintained. Meanwhile, once soft particles in the composition are captured on the filter surface, the soft particles are deformed in accordance with the flow of the composition. Eventually, gaps among the soft particles disappear, making it difficult for the composition to pass through the filter. Hence, the ability to purify the composition is decreased. When this condition further continues, the filter consequently clogs, and the filter needs to be replaced. When such filter replacement is repeated, filters for producing such a composition are consumed in uneconomically large quantities.

In this event, when the difference in the hydraulic pressure before and after the filter is set greater than 50 KPa in order to guarantee the passage amount of the composition, foreign matters, particularly soft particles, which ought to be captured by the filter, pass through pores of the filter and enter the filtrate side by the pressure difference. Thereby, the purification by filtration becomes insufficient. This causes a large number of defects in coating or after etching, decreasing the yield in manufacturing a semiconductor device.

WO 2017/038968 discloses the purification of dihydroxynaphtalene by mixing with propylene glycol monomethyl ether (PGME) and filtering the mixture using a polyamide hollow fiber membrane filter.

### CITATION LIST

### PATENT LITERATURE

Patent Literature 1: Japanese Unexamined Patent Application Publication No. 2016-75937

### SUMMARY OF INVENTION

### TECHNICAL PROBLEM

The present invention has been made in view of the above problems. An object of the present invention is to provide a method for purifying dihydroxynaphthalene, the method making it possible to suppress soft particle generation and obtain a dihydroxynaphthalene serving as a raw material of a resin and composition excellent in filterability.

### SOLUTION TO PROBLEM

To accomplish the above object, the present invention provides a method for purifying dihydroxynaphthalene, comprising a step of
removing a sulfur content in the dihydroxynaphthalene with an adsorbent, wherein
neutral alumina is used as the adsorbent.

Such an inventive method for purifying dihydroxynaphthalene suppresses soft particle generation and makes it possible to obtain dihydroxynaphthalene having a low sulfur content and serving as a raw material of a resin and composition excellent in filterability (hereinafter referred to as purified dihydroxynaphthalene).

Preferably, the dihydroxynaphthalene is dissolved into an organic solvent, the neutral alumina is added to the solution and stirred, and the neutral alumina is separated by filtration.

Such a purification method makes it possible to more easily obtain purified dihydroxynaphthalene serving as a raw material of a resin and composition excellent in filterability.

Moreover, preferably, the neutral alumina is added in an amount of 5 parts by mass or more relative to 100 parts by mass of the dihydroxynaphthalene, and the stirring is performed at a temperature of 0 to 150°C for 0.1 hours or more.

Such a purification method can more surely remove the sulfur content, suppress soft particle generation, and obtain purified dihydroxynaphthalene serving as a raw material of a resin and composition excellent in filterability.

Further, the dihydroxynaphthalene to be purified is preferably 1,5-dihydroxynaphthalene or 2,7-dihydroxynaphthalene.

The inventive method for purifying dihydroxynaphthalene can be particularly suitably employed for 1,5-dihydroxynaphthalene and 2,7-dihydroxynaphthalene.

In addition, a sulfur element content among constituent elements contained in the purified dihydroxynaphthalene is preferably 100 ppm or less in terms of mass.

Furthermore, the sulfur element content among the constituent elements contained in the purified dihydroxynaphthalene is preferably 50 ppm or less in terms of mass.

With such sulfur element contents, a composition containing a resin produced by using the purified dihydroxynaphthalene is more reliably subjected to purification by precise filtration with a filter having openings of 20 nm or less, which is essential for the most advanced processing material for manufacturing a semiconductor device.

### ADVANTAGEOUS EFFECTS OF INVENTION

As described above, the inventive method for purifying dihydroxynaphthalene enables effective removal of a sulfonic acid compound which results in soft particles. This makes it possible to obtain dihydroxynaphthalene which serves as a raw material of a resin and composition excellent in filterability.

### BRIEF DESCRIPTION OF DRAWINGS

FIG. 1 is a graph in which amounts of polymers, which were produced in Synthesis Example 1 and Comparative Synthesis Example 1, filtered through a filter over time are plotted.
FIG. 2 shows photographs of observing the filter with SEM after the filtration of the polymer produced in Comparative Synthesis Example 1.

### DESCRIPTION OF EMBODIMENTS

As has been described above, there have been demands for developments of a purification method capable of suppressing soft particle generation and obtaining dihydroxynaphthalene serving as a raw material of a resin and composition excellent in filterability.

As described above, if a composition for forming an organic film contains soft particles, the soft particles decrease the productivity in the process of producing the composition for forming an organic film, or decrease the yield of the semiconductor manufacturing apparatus in some cases. Thus, soft particle generation needs to be prevented.

Hence, the present inventors have earnestly studied to achieve the above-described object and consequently found that the use of neutral alumina as an adsorbent to remove a sulfonic acid compound contained in a starting material dihydroxynaphthalene makes it possible to suppress soft particle generation. Moreover, the inventors have found that when a composition containing a resin using purified dihydroxynaphthalene is produced, purification is possible by precise filtration with a filter having openings of 20 nm or less, which is essential for the most advanced processing material for manufacturing a semiconductor device. These findings have led to the completion of the present invention.

Specifically, the present invention is a method for purifying dihydroxynaphthalene, comprising a step of removing a sulfur content in the dihydroxynaphthalene with an adsorbent, wherein neutral alumina is used as the adsorbent.

Hereinafter, embodiments of the present invention will be described in detail. However, the present invention is not limited thereto.

The dihydroxynaphthalene usable in the inventive purification method is not particularly limited. Examples thereof include 1,2-dihydroxynaphthalene, 1,3-dihydroxynaphthalene, 2,3-dihydroxynaphthalene, 1,4-dihydroxynaphthalene, 1,5-dihydroxynaphthalene, 1,6-dihydroxynaphthalene, 2,6-dihydroxynaphthalene, 1,7-dihydroxynaphthalene, 2,7-dihydroxynaphthalene, 1,8-dihydroxynaphthalene, and the like. The dihydroxynaphthalene is particularly preferably 1,5-dihydroxynaphthalene or 2,7-dihydroxynaphthalene.

As described above, the dihydroxynaphthalene (1) is industrially produced by the following method in general. Specifically, the starting material naphthalene (1-1) is sulfonated to obtain the sulfonic acid compound (1-2). Then, this compound is converted to have hydroxyl groups by alkali fusion, so that the dihydroxynaphthalene (1) is obtained. 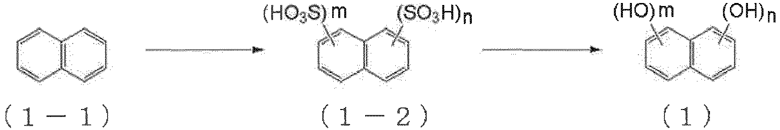 In the formulae, "m" and "n" are as defined above.

In this event, the sulfonic acid compound (1-2) is not completely consumed by the alkali fusion. As the sulfur element content among the constituents elements, the sulfonic acid compound (1-2) remains in an amount of approximately several hundred ppm to several thousand ppm in terms of mass in the dihydroxynaphthalene (1) of general industrial grades. Table 1 below shows contents, in terms of mass, of sulfur elements contained in dihydroxynaphthalenes of general industrial grades.

**[Table 1]**

| | Company A-1 | Company A-2 | Company A-3 | Company B-1 | Company B-2 | Company C-1 | Company C-2 | Company C -3 |
|---|---|---|---|---|---|---|---|---|
| Sulfur element content/ppm | 1390 | 5300 | 260 | 1390 | 5300 | 260 | 260 | 300 |

Note that, as the method for quantifying the sulfur element in dihydroxynaphthalene (1), there are known a combination method of sample combustion and titration, a combination method of sample combustion and ion chromatography, and inductively coupled plasma emission spectroscopy (ICP-AES/OES), and the like. Among these, more highly sensitive ICP-AES/OES is preferable.

In the present invention, an adsorption treatment is performed with neutral alumina to remove the sulfonic acid compound (1-2) which is an impurity contained in industrial-grade dihydroxynaphthalene. When the adsorption treatment is performed with neutral alumina, an adsorbent other than the neutral alumina may be mixed for use, such as activated carbon, acidic alumina, or basic alumina.

The neutral alumina used in the present invention is preferably granules whose hue is pale yellow to white, more preferably white granules. Moreover, the aluminum oxide purity is preferably 85% or more, more preferably 94.0% or more. The ignition loss of the adsorbent portion is preferably 8% or less, more preferably 5.5% or less. The adsorbent has a bulk density of preferably 8 to 20 ml/10 g, more preferably 12 to 16 ml/10 g. Further, the activated alumina has a pH of preferably 6.5 to 8.5, more preferably 7.0 to 8.0.

In the particle size distribution of the neutral alumina used as the adsorbent, preferably, particles of 63 µm to 250 µm account for 80% or more, and particles of less than 63 µm account for less than 10%. More preferably, particles of less than 60 µm account for less than 5% so as to facilitate the process of removing the neutral alumina after the adsorption.

Moreover, the method for using the neutral alumina as the adsorbent is not particularly limited. In a preferable method, for example, the dihydroxynaphthalene is dissolved into an organic solvent, the neutral alumina is added as the adsorbent to the solution and stirred, and the neutral alumina is separated by filtration.

In this event, the organic solvent for dissolving the dihydroxynaphthalene before purification is not particularly limited. Examples of the organic solvent include alcohols such as methanol, ethanol, propanol, propylene glycol methyl ether, methyl cellosolve, and ethyl cellosolve; ketones such as methyl ethyl ketone, methyl isobutyl ketone, methyl amyl ketone, and cyclohexanone; esters such as ethyl acetate, propylene glycol methyl ether acetate, and γ-butyrolactone; aliphatic hydrocarbons such as pentane and hexane; aromatic hydrocarbons such as toluene and xylene; ethers such as tetrahydrofuran and dioxane; and the like.

The neutral alumina is preferably added in an amount of 5 parts by mass or more relative to 100 parts by mass of the dihydroxynaphthalene before purification. When such an amount of the neutral alumina is added, the sulfur content in the dihydroxynaphthalene can be surely removed. Although the upper limit is not particularly limited, it is not economical to use the neutral alumina in a large amount. Thus, the use of 100 parts by mass of the neutral alumina is sufficient.

In the above-described method, after the neutral alumina is added, the solution is stirred at a temperature of preferably 0 to 150°C. Moreover, the stirring time is preferably 0.1 hours or more. The upper limit of the stirring time is not particularly limited, and the stirring for 20 hours is sufficient.

The sulfur element content among constituent elements contained in the purified dihydroxynaphthalene obtained by the present invention is preferably 100 ppm or less, more preferably 50 ppm or less, in terms of mass. The sulfur element content among the constituent elements contained in the purified dihydroxynaphthalene is preferably 100 ppm or less in terms of mass because soft particle formation is suppressed in a composition containing a resin produced by using such a purified dihydroxynaphthalene and because purification is surely possible by precise filtration with a filter having openings of 20 nm or less, which is essential for the most advanced processing material for manufacturing a semiconductor device.

Such an inventive method for purifying dihydroxynaphthalene is capable of effectively removing a sulfonic acid compound which causes soft particles, and accordingly makes it possible to obtain dihydroxynaphthalene which is polymerized into a resin having excellent filterability.

### EXAMPLE

Hereinafter, the present invention will be specifically described by referring to Examples and Comparative Examples. However, the present invention is not limited to these descriptions. Note that, as molecular weights, weight average molecular weight (Mw) and number average molecular weight (Mn) were measured in terms of polystyrene by gel permeation chromatography (GPC). Then, the dispersity (Mw/Mn) was calculated therefrom.

### (Example 1-1)

In a solvent mixture containing 127.6 g of propylene glycol methyl ether (hereinafter, PGME) and 353.6 g of methyl isobutyl ketone (hereinafter, MIBK), 72.2 g of commercially available 1,5-dihydroxynaphthalene (hereinafter, 15DHN, sulfur element content: 400 ppm, measured by ICP-OES) was dissolved. To this, 57.2 g of neutral alumina (manufactured by Tomita Pharmaceutical Co., Ltd., Tomita-AD-250NS, particle diameters: 60 to 250 µm, pH: 7.5) was added and stirred at room temperature for 12 hours. Then, the neutral alumina was separated by filtration, and the resulting 15DHN solution in PGME/MIBK was washed with ion-exchanged water. PGME was further added and concentrated. Thus, 500 g of a 13 wt% 15DHN-PGME solution was obtained (purified raw material 1). The sulfur element content in the solid content of this 15DHN solution was measured by ICP-OES and found to be 45 ppm.

### (Comparative Example 1-1)

A comparative purified raw material 1 was obtained by the purification according to the same reactions in Example 1-1, except that silica gel (manufactured by Kanto Chemical Co., Inc., Silica gel 60, particle diameters: 63 Lo 210 µm, pore diameters: 6.5 µm, pH: 6.0) was used in place of the neutral alumina.

### (Comparative Example 1-2)

A comparative purified raw material 2 was obtained by the purification according to the same reactions in Example 1-1, except that acidic alumina (manufactured by Wako Pure Chemical Industries, Ltd., MP Alumina, Activated, Acidic, Super I, particle diameters: 50-200 µm, pH: 4.5) was used in place of the neutral alumina.

### (Comparative Example 1-3)

A comparative purified raw material 3 was obtained by the purification according to the same reactions in Example 1-1, except that basic alumina (manufactured by Wako Pure Chemical Industries, Ltd., MP Alumina, Activated, Basic, Activity: I, particle diameters: 50-200 µm, pH: 9.0) was used in place of the neutral alumina.

### (Comparative Example 1-4)

A comparative purified raw material 4 was obtained by the purification according to the same reactions in Example 1-1, except that activated carbon (manufactured by KURARAY CO., LTD., GLC, particle diameters: 72 µm, pores: 2.8 nm) was used in place of the neutral alumina.

### (Comparative Example 1-5)

A comparative purified raw material 5 was obtained by the purification according to the same reactions in Example 1-1, except that activated carbon (manufactured by Japan EnviroChemicals, Ltd, SHIRASAGI M, particle diameters: 75 µm, pores: 2.0 nm) was used in place of the neutral alumina.

### (Comparative Example 1-6)

A comparative purified raw material 6 was obtained by the purification according to the same reactions in Example 1-1, except that a cation-exchange resin (manufactured by Mitsubishi Chemical Corporation, DIAION SK110, strongly acidic sulfone-based ion-exchange resin) was used in place of the neutral alumina.

### (Comparative Example 1-7)

A comparative purified raw material 7 was obtained by the purification according to the same reactions in Example 1-1, except that an anion-exchange resin (manufactured by Mitsubishi Chemical Corporation, DIAION SA10A, strongly basic quaternary ammonium-based ion-exchange resin) was used in place of the neutral alumina.

### [Method for measuring sulfur element content by ICP-OES]

### (Pretreatment: microwave digestion method)

These samples (the purified raw material 1, the comparative purified raw materials 1 to 7) were pretreated for ICP-OES by using a microwave sample digestion system (MULTIWAVE 3000 manufactured by Anton Paar GmbH). 0.5 g of each sample and 10 ml of 69.0% nitric acid (Ultrapur-100 manufactured by Kanto Chemical Co., Inc.) were put into a PTFE (polytetrafluoroethylene) digestion container and left standing for 1 hour. Then, microwave digestion was carried out with this system for 85 minutes. After the digestion, the solution was transferred to a TPX storage container and diluted with distilled water such that the total amount became 50 g. Thus, 100-fold diluted samples were prepared.

### (ICP-OES measurement)

The analysis of the sulfur element content by ICP-OES was conducted with a CID/ICP emission spectrometer (iCAP6000DUO manufactured by Thermo Fisher SCIENTIFIC Inc.). The pretreated 100-fold diluted samples described above were measured under the following setting conditions. Table 2 below shows the measured sulfur element contents.

### (ICP-OES setting conditions)

High-frequency output: 1150 W
Plasma gas flow rate: 12.0 L/min
Auxiliary gas flow rate: 0.5 L/min
Nebulizer gas flow rate: 0.5 L/min
Peristaltic pump rotational speed: 50 rpm
Measurement direction: axial
Analysis integration times (low wavelength/high wavelength): 10 seconds/10 seconds
The number of analysis integrations: 3 times
Sample replacement time: 30 seconds

**[Table 2]**

| | | Adsorbent | Sulfur element content (ppm) |
|---|---|---|---|
| Example 1-1 | purified raw material 1 | neutral alumina | 45 |
| Comparative Example 1-1 | comparative purified raw material 1 | silica gel | 380 |
| Comparative Example 1-2 | comparative purified raw material 2 | acidic alumina | 370 |
| Comparative Example 1-3 | comparative purified raw material 3 | basic alumina | 300 |
| Comparative Example 1-4 | comparative purified raw material 4 | activated carbon (pores: 2.8 nm) | 350 |
| Comparative Example 1-5 | comparative purified raw material 5 | activated carbon (pores: 2.0 nm) | 200 |
| Comparative Example 1-6 | comparative purified raw material 6 | cation-exchange resin | 390 |
| Comparative Example 1-7 | comparative purified raw material 7 | anion-exchange resin | 290 |

### (Synthesis Example 1)

In a 1000-ml flask, 500 g (0.5 moles) of the 13 wt% 15DHN-PGME solution (purified raw material 1) purified in Example 1-1 was mixed with 2.8 g of p-toluenesulfonic acid and 2.8 g of PGME. While the mixture was being stirred at 80°C, 14.3 g of a 50 wt% formaldehyde aqueous solution was added thereto. With the temperature kept at 80°C, the stirring continued for 6 hours. Then, the temperature was cooled to room temperature (monomer conversion ratio: 77%). The resulting solution was concentrated under reduced pressure. Subsequently, 540 g of hexane was added thereto, and the polymer content was separated and precipitated. The upper filtrate was separated by filtration and removed. Thereafter, this operation was repeated, so that the residual monomer content was 5% or less. The remaining polymer content was dissolved again in 200 g of MIBK, 200 g of ion-exchanged water was added thereto, and the metal ion content was removed. To the MIBK solution from which the metal ion content had been removed, 300 g of propylene glycol monomethyl ether acetate (PGMEA) was added and concentrated under reduced pressure. Thus, a PGMEA solution containing approximately 20 wt% of a polymer 1 as shown by the following formula was obtained. The molecular weight (Mw) and the dispersity (Mw/Mn) of the obtained polymer in terms of polystyrene were measured by gel permeation chromatography (GPC). The sulfur element content was measured by ICP-OES. As a result, the molecular weight (Mw) was 3,500, the dispersity (Mw/Mn) was 2.01, and the sulfur element content in the solid content was 45 ppm. 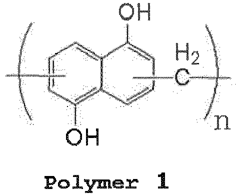 In the formula, "n" represents the number of repeating units, ranging from 2 to 100.

### (Comparative Synthesis Example 1)

A comparative polymer 1 was obtained according to the same reactions in Synthesis Example 1, except that the commercially available 15DHN was not treated with the neutral alumina but was used for the polymerization reaction. The molecular weight (Mw) and the dispersity (Mw/Mn) of the obtained comparative polymer 1 in terms of polystyrene were measured by gel permeation chromatography (GPC). The sulfur element content was measured by ICP-OES. As a result, the molecular weight (Mw) was 3,600, the dispersity (Mw/Mn) was 2.03, and the sulfur element content was 435 ppm.

### [Polymer (dihydroxynaphthalene resin) filtration study 1]

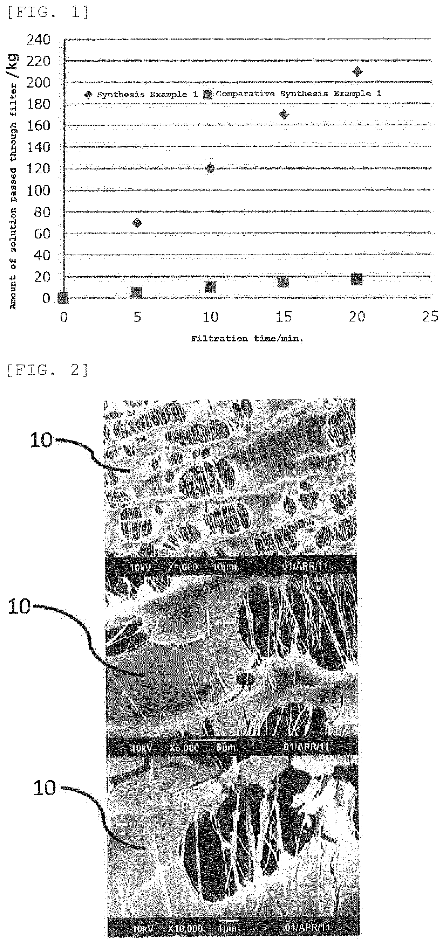

PGMEA solutions containing 20 mass% of the polymer 1 (neutral alumina-treated product) obtained in Synthesis Example 1 or the comparative polymer 1 (neutral alumina-untreated product) obtained in Comparative Synthesis Example 1 were prepared and filtered through a 10-inch PTFE filter having 0.1-µm openings. The relationship between the filtration time and the weight of the solution passed was as shown in FIG. 1. It was verified that the filterability of the comparative polymer 1 was significantly poor while the filterability of the polymer 1 was favorable.

In addition, the top of the filter where the comparative polymer 1 was poorly filtered was observed with SEM. It was found as shown in FIG. 2 that a component of a viscous material 10 (soft particles) adhered on the filter.

Presumably, in the comparative polymer 1, sulfonic acid impurities as shown below were incorporated into the polymer, thereby forming the filter deposit which deteriorated the filterability. 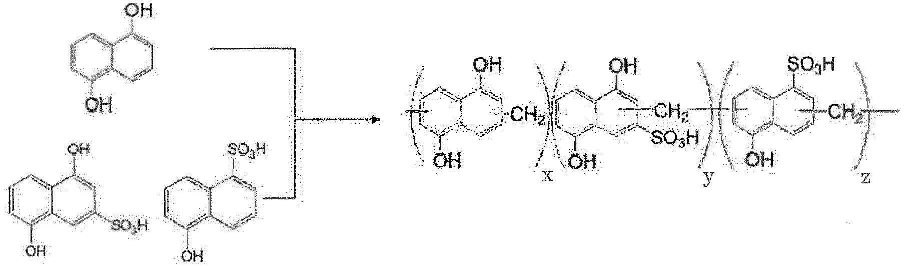 In the formula, "x", "y", and "z" each represent the number of repeating units. x+y+z ranges from 2 to 100.

### [Polymer (dihydroxynaphthalene resin) filtration study 2]

PGMEA solutions containing 20 mass% of the polymer 1 (neutral alumina-treated product) obtained in Synthesis Example 1 or the comparative polymer 1 (neutral alumina-untreated product) obtained in Comparative Synthesis Example 1 were prepared and filtered through a nylon filter having 20-nm openings. The polymer 1 was successfully filtered at the filtration pressure of 50 kPa. In contrast, the filter was clogged with the comparative polymer 1. Even when the pressure was increased to 500 kPa, no filtrate was obtained.

From the foregoing, it was revealed that in Example 1-1 using the neutral alumina to remove the sulfur content contained in dihydroxynaphthalene, the sulfur element was removed such that the resulting content was 100 ppm or less in terms of mass. In contrast, it was revealed that in Comparative Examples 1-1 to 1-7 using the other adsorbents than the neutral alumina, the sulfur contents were not removed as much as that in the present invention. In addition, as in Synthesis Example 1, the resin using the purified dihydroxynaphthalene from which the sulfur content had been removed had favorable filterability. Meanwhile, the resin using the dihydroxynaphthalene from which the sulfur content had not been removed as in Comparative Synthesis Example 1 had poor filterability.

### [Preparation of composition for forming organic underlayer film (dihydroxynaphthalene composition)]

Next, the polymer 1, the comparative polymer 1, and various additives were blended into compositions shown in Table 3 below (the units in the parentheses are kilogram), and filtered through a nylon filter having a 10 inch size with 20-nm openings by using a production facility for a composition for forming an organic underlayer film. Thus, compositions for forming an organic underlayer film were produced (SOL-1, -2, comparative SOL-1, -2).

**[Table 3]**

| SOL | Polymer (kg) | Crosslinking agent (kg) | Acid generator (kg) | Surfactant (kg) | Solvent (kg) |
|---|---|---|---|---|---|
| SOL-1 | polymer 1 (1) | - | - | SF1 (0.005) | PGMEA (25) |
| SOL-2 | polymer 1 (1) | CL1 (0.1) | AG1 (0.01) | SF1 (0.005) | PGMEA (25) |
| comparative SOL-1 | comparative polymer 1 (1) | - | - | SF1 (0.5) | PGMEA (25) |
| comparative SOL-2 | comparative polymer 1 (1) | CL1 (0.1) | AG1 (0.01) | SF1 (0.005) | PGMEA (25) |

The crosslinking agent CL1, the acid generator AG1, and the surfactant SF1 used were as follows. 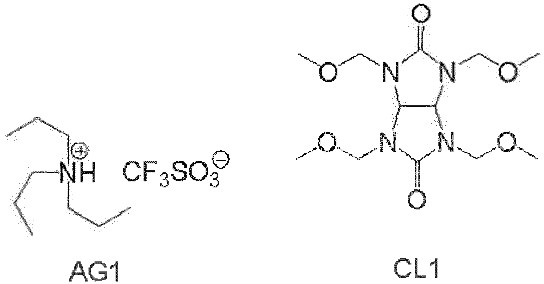 SF1: FC-4430 manufactured by 3M

The flow speeds during the filtration with the filter and the pressure differences between before and after the filter are shown in Table 4.

**[Table 4]**

| Composition | SOL | Sulfur content in condensate | Flow speed during filtration | Pressure difference between before and after filter |
|---|---|---|---|---|
| UL1-1 | SOL-1 | 45 ppm | 300 g/min. | 11 KPa |
| UL1-2 | SOL-1 | 45 ppm | 800 g/min. | 30 KPa |
| UL2-1 | SOL-2 | 45 ppm | 300 g/min. | 10 KPa |
| UL2-2 | SOL-2 | 45 ppm | 800 g/min. | 29 KPa |
| comparative UL1-1 | comparative SOL-1 | 435 ppm | 300 g/min. | 40 KPa |
| comparative UL1-2 | comparative SOL-1 | 435 ppm | 800 g/min. | 100 KPa |
| comparative UL2-1 | comparative SOL-2 | 435 ppm | 300 g/min. | 39 KPa |
| comparative UL2-2 | comparative SOL-2 | 435 ppm | 800 g/min. | 100 KPa |

Each of the obtained compositions for forming an organic underlayer film was connected to Clean Track ACT12 manufactured by Tokyo Electron Limited, and applied onto a 12-inch (diameter: 300 mm) silicon wafer with no filter being connected to the connection pipe. The resultant was baked at 250°C for 60 seconds to prepare a coating film. A defect with a size of 60 nm or more on the coating film was checked by defect inspection using a dark-field defect inspection system SP5 manufactured by KLA-Tencor Corporation (Examples 2-1 to 2-4, Comparative Examples 2-1 to 2-4). Table 5 below shows the result.

**[Table 5]**

| Example | Composition | Number of defects |
|---|---|---|
| Example 2-1 | UL1-1 | 10 |
| Example 2-2 | UL1-2 | 20 |
| Example 2-3 | UL2-1 | 12 |
| Example 2-4 | UL2-2 | 26 |
| Comparative Example 2-1 | comparative UL1-1 | 313 |
| Comparative Example 2-2 | comparative UL1-2 | 538 |
| Comparative Example 2-3 | comparative UL2-1 | 298 |
| Comparative Example 2-4 | comparative UL2-2 | 657 |

These results revealed that, as in Examples 2-1 to 2-4, the use of the dihydroxynaphthalene resin obtained by condensing the purified dihydroxynaphthalene, which was purified according to the inventive purification method, reduced the number of defects in the coating film prepared from the obtained composition. In contrast, as in Comparative Examples 2-1 to 2-4, when dihydroxynaphthalene not purified unlike the present invention was used, the resulting dihydroxynaphthalene resin had a sulfur element content exceeding 100 ppm in terms of mass; if this dihydroxynaphthalene resin was used, the pressure difference between before and after the filter was increased when the composition was filtered and purified. Thus, foreign matters in the composition were hardly trapped by the filter, and the inspection of the coating film prepared from the obtained composition showed a large number of defects in the coating film.

From the foregoing, it was revealed that the present invention suppresses soft particle generation from sulfur content, making it possible to obtain dihydroxynaphthalene serving as a raw material of a resin and composition excellent in filterability.

It should be noted that the present invention is not restricted to the above-described embodiments. The embodiments are merely examples so that any embodiments that have substantially the same feature and demonstrate the same functions and effects as those in the technical concept as disclosed in claims of the present invention are included in the technical range of the present invention.

## Claims

1. A method for purifying dihydroxynaphthalene, comprising a step of
removing a sulfur content in the dihydroxynaphthalene with an adsorbent, wherein
neutral alumina is used as the adsorbent.

2. The method for purifying dihydroxynaphthalene according to claim 1, wherein
the dihydroxynaphthalene is dissolved into an organic solvent,
the neutral alumina is added to the solution and stirred, and
the neutral alumina is separated by filtration.

3. The method for purifying dihydroxynaphthalene according to claim 2, wherein
the neutral alumina is added in an amount of 5 parts by mass or more relative to 100 parts by mass of the dihydroxynaphthalene, and
the stirring is performed at a temperature of 0 to 150°C for 0.1 hours or more.

4. The method for purifying dihydroxynaphthalene according to any one of claims 1 to 3, wherein the dihydroxynaphthalene to be purified is 1,5-dihydroxynaphthalene or 2,7-dihydroxynaphthalene.

5. The method for purifying dihydroxynaphthalene according to any one of claims 1 to 4, wherein a sulfur element content among constituent elements contained in the purified dihydroxynaphthalene is 100 ppm or less in terms of mass.

6. The method for purifying dihydroxynaphthalene according to claim 5, wherein the sulfur element content among the constituent elements contained in the purified dihydroxynaphthalene is 50 ppm or less in terms of mass.

## Patentansprüche

1. Verfahren zur Reinigung von Dihydroxynaphthalen, umfassend einen Schritt der
Entfernung eines Schwefelgehalts in dem Dihydroxynaphthalen mit einem Adsorbens, wobei neutrales Alumina als Adsorbens verwendet wird.

2. Verfahren zur Reinigung von Dihydroxynaphthalen gemäß Anspruch 1, wobei
das Dihydroxynaphthalen in einem organischen Lösemittel aufgelöst wird,
das neutrale Alumina wird der Lösung zugegeben und es wird gerührt, und
das neutrale Alumina wird durch Filtration abgetrennt.

3. Verfahren zur Reinigung von Dihydroxynaphthalen gemäß Anspruch 2, wobei
das neutrale Alumina in einer Menge von 5 Massenteilen oder mehr, relativ zu 100 Massenteilen des Dihydroxynaphthalens, zugegeben wird, und
Rühren wird durchgeführt bei einer Temperatur von 0 bis 150°C über 0,1 Stunden oder darüber.

4. Verfahren zur Reinigung von Dihydroxynaphthalen gemäß irgendeinem der Ansprüche 1 bis 3, wobei das zu reinigende Dihydroxynaphthalen 1,5-Dihydroxynaphthalen oder 2,7-Dihydroxynaphthalen ist.

5. Verfahren zur Reinigung von Dihydroxynaphthalen gemäß irgendeinem der Ansprüche 1 bis 4, wobei ein Schwefelelementgehalt unter den konstituierenden Elementen, die in dem gereinigten Dihydroxynaphthalen enthalten sind, 100 ppm oder darunter ist, angegeben als Masse.

6. Verfahren zur Reinigung von Dihydroxynaphthalen gemäß Anspruch 5, wobei der Schwefelelementgehalt unter den konstituierenden Elementen, die in dem gereinigten Dihydroxynaphthalen enthalten sind, 50 ppm oder darunter ist, angegeben als Masse.

## Revendications

1. Procédé de purification de dihydroxynaphtalène, comprenant une étape
d'élimination d'une teneur en soufre dans le dihydroxynaphtalène avec un adsorbant, dans laquelle
de l'alumine neutre est utilisée comme adsorbant.

2. Procédé de purification de dihydroxynaphtalène selon la revendication 1, dans lequel
le dihydroxynaphtalène est dissous dans un solvant organique,
l'alumine neutre est ajoutée à la solution et agitée, et
l'alumine neutre est séparée par filtration.

3. Procédé de purification de dihydroxynaphtalène selon la revendication 2, dans lequel
l'alumine neutre est ajoutée en une quantité de 5 parties en masse ou plus par rapport à 100 parties en masse du dihydroxynaphtalène, et
l'agitation est effectuée à une température de 0 à 150 °C pendant 0,1 heure ou plus.

4. Procédé de purification de dihydroxynaphtalène selon l'une quelconque des revendications 1 à 3, dans lequel le dihydroxynaphtalène à purifier est le 1,5-dihydroxynaphtalène ou le 2,7-dihydroxynaphtalène.

5. Procédé de purification de dihydroxynaphtalène selon l'une quelconque des revendications 1 à 4, dans lequel une teneur en éléments soufrés parmi les éléments constitutifs contenus dans le dihydroxynaphtalène purifié est de 100 ppm ou moins en termes de masse.

6. Procédé de purification de dihydroxynaphtalène selon la revendication 5, dans lequel la teneur en éléments soufrés parmi les éléments constitutifs contenus dans le dihydroxynaphtalène purifié est de 50 ppm ou moins en termes de masse.
